(19) Europäisches Patentamt
European Patent Office
Office européen des brevets

(11) Publication number: **0 271 212 B1**

(12) **EUROPEAN PATENT SPECIFICATION**

(45) Date of publication of patent specification: **16.09.92**

(51) Int. Cl.5: **C07C 67/10**, C07C 69/63, C07C 69/65

(21) Application number: **87309881.8**

(22) Date of filing: **09.11.87**

(54) **Process for preparation of carboxylic acid ester containing fluorine.**

(30) Priority: **07.11.86 JP 264088/86
12.05.87 JP 113460/87
15.05.87 JP 116950/87
17.07.87 JP 177147/87
23.07.87 JP 182291/87**

(43) Date of publication of application:
**15.06.88 Bulletin 88/24**

(45) Publication of the grant of the patent:
**16.09.92 Bulletin 92/38**

(84) Designated Contracting States:
**CH DE FR GB IT LI**

(56) References cited:
**DE-C- 1 264 444**

**PATENT ABSTRACT OF JAPAN, vol. 10, no. 235 (C-366)(2291) 14th August 1986; & JP-A-61 69742**

**PATENT ABSTRACTS OF JAPAN, vol. 7, no. 273 (C-198)(1418), 6th December 1983; & JP-A-58 154 529**

(73) Proprietor: **Tosoh Corporation
4560, Kaisei-cho
Shinnanyo-shi, Yamaguchi-ken(JP)**

(72) Inventor: **Shuyama, Hideo
4073-10, Oaza Mure
Hofu-shi Yamaguchi-ken(JP)**
Inventor: **Ogawa, Tsukasa
2591, Oaza Tonda
Shinnanyo-shi Yamaguchi-ken(JP)**
Inventor: **Takahashi, Mitsuru
3056-1, Oaza Tonda
Shinnanyo-shi Yamaguchi-ken(JP)**
Inventor: **Hamada, Michiyuki
2591, Oaza Tonda
Shinnanyo-shi Yamaguchi-ken(JP)**
Inventor: **Yoshimitsu, Mitsuaki
1-12-41, Nomura
Shinnanyo-shi Yamaguchi-ken(JP)**
Inventor: **Oyama, Kiyotaka
7-15-12, Nijigaoka
Hikari-shi Yamaguchi-ken(JP)**

(74) Representative: **Sheard, Andrew Gregory et al
Kilburn & Strode 30, John Street
London WC1N 2DD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

PATENT ABSTRACTS OF JAPAN, vol. 7, no. 248 (C-193)(1393), 4th November 1983; & JP-A-58 135 836

HOUBEN-WEYL, "Methoden der organischen Chemie", Section E5, pp. 470-471 and 684-690 (1985)

## Description

The present invention relates to a process for the preparation of a carboxylic acid ester containing fluorine, from an organic halide containing fluorine.

Carboxylic acid esters containing fluorine show good characteristics in their chemical, physical and physiological properties. They are important raw materials for polymers from which may be produced specific surfactants, water and oil repellants, and intermediates for synthetic medical and agricultural chemicals and also for polymers used for painting materials, fluorine rubbers, resist materials, contact lenses and other plastic optical materials.

Carboxylic acid esters containing fluorine have previously been prepared by various methods including processes employing a carboxylic acid containing fluorine. Examples of processes of the type used in the prior art include:

(1) A method in which a carboxylic acid containing fluorine is converted into a corresponding acid halide and reacted with an alcohol (J. Am. Chem. Soc., 75, 966(1953)).

(2) A method in which a carboxylic acid containing fluorine is reacted with an alcohol in the presence of an acid catalyst (J. Am. Chem. Soc., 73, 4625(1951)).

(3) A method in which a fluoroaliphatic carboxylic acid is reacted with diazomethane to prepare a methyl ester.

Further examples of methods used in the prior art to prepare carboxylic acid esters containing fluorine include:

(4) Thermal decomposition of 2-trifluoromethyl-2-acetoxypropionic acid ester (at 500 ° C) (J. Chem. Soc., 1954, 366), and

(5) 2-Bromo-3,3,3-trifluoropropene is reacted with carbon monoxide in the presence of an alcohol using a palladium catalyst to obtain an ester (Japanese Laid-Open Patent Application No. Sho. 58-154529).

Among these processes of synthesis in the prior art methods (1) to (3) utilise carboxylic acid containing fluorine as an obligatory starting material. The carboxylic acid containing fluorine needs to be synthesised since it occurs rarely in nature. Therefore, if these processes according to the prior art are to be employed to produce a carboxylic acid ester containing fluorine, a corresponding carboxylic acid containing fluorine has to be prepared by methods such as, for example, electrolytic fluorination of a carboxylic acid halide and preparation of a carboxylic acid starting from an organic halide compound containing fluorine, followed by isolation of the product and transformation into an ester. Thus, the methods require two or more steps of reaction procedure.

The methods (4) and (5) are not free from problems such as difficulty in preparing the starting materials, low yield, and toxicity of the starting materials.

In short, the conventional methods related to preparation of carboxylic acid ester containing fluorine are associated with the problems that the yield is low, conditions of the reactions are severe and the procedure of the reaction is complicated.

Taking the above-mentioned situation into account, the present inventors carried out their investigations, aiming to establish a process for the production of a carboxylic acid ester containing fluorine which is easy to follow in practice and industrially applicable.

One of the known methods for producing an organic carboxylic acid containing fluorine starting from a fluorine containing halogen compound, is a method in which the halogen compound and carbon dioxide react in the presence of zinc, followed by treatment of the reaction product with acid to obtain the carboxylic acid.

Instead of treating the reaction product with acid to obtain a carboxylic acid which could subsequently be treated to form a carboxylic acid ester, as discussed above, the present inventors investigated reacting the reaction mixture directly with a halogenated hydrocarbon.

It was found by the present inventors that the reaction mixture afforded, on reaction directly with a halogenated hydrocarbon, a surprisingly high yield of a carboxylic acid ester containing fluorine.

Thus, according to the present invention, there is provided a process for the preparation of a carboxylic acid ester containing fluorine wherein the process comprises the reaction of a halogenated hydrocarbon represented by the general formula (A)

(A) RY

where R represents
a straight or branched chained alkyl group having 1 to 20 carbon atoms which optionally contains fluorine,
a saturated or unsaturated straight or branched chained aliphatic group having up to 20 carbon atoms which

is substituted by an aromatic ring,
or an alkenyl group represented by a general formula

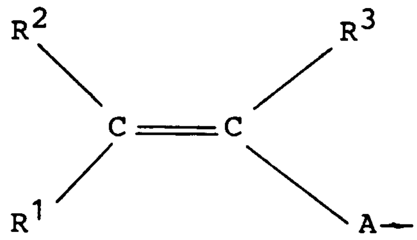

where $R^1$, $R^2$ and $R^3$ independently represent a hydrogen atom
or a straight or branched chain alkyl group
and A represents a methylene group or a straight or branched chain alkylene group having 3 to 20 carbon atoms
and Y represents a chlorine atom, a bromine atom or an iodine atom
with a mixture obtainable by the reaction of an organic halogenated compound containing fluorine represented by the formula (B), with carbon dioxide in the presence of zinc:

(B) $X - R_f - X'$

where X and X′ independently represent
fluorine, chlorine, bromine, or iodine atoms
with the proviso that X and X′ may not both represent fluorine atoms
and $R_f$ represents a saturated or unsaturated aliphatic group which is straight or branched chained
either having 1 to 20 carbon atoms when X or X′ represents a fluorine atom
or having 3 to 20 carbon atoms when neither X nor X′ represents a fluorine atom.

Preferably, the mixture obtainable by the reaction of the organic halogen compound containing fluorine represented by the formula (B) with carbon dioxide in the presence of zinc is so obtained.

It is preferred that the reaction of the organic halogenated compound containing fluorine with carbon dioxide in the presence of zinc is carried out in the presence of one or more of the following cations, namely, alkali metal ions, alkaline earth ions, or ammonium ions.

Other preferred process features include the performance of the process in an aprotic polar organic solvent, and performance of the reaction of the halogenated hydrocarbon with the reaction product in a temperature range from 0 ° C to 300 ° C.

The organic halogenated compound containing fluorine used in the present invention encompasses a number of groups of compounds.

One preferred group is that in which either X or X′ represents a fluorine atom in the organic halogenated compound containing fluorine. In the reaction of the organic halogenated compound containing fluorine with carbon dioxide in the presence of zinc it is the X or X′ which is other than fluorine which alone reacts in order to give an ultimate product, after reaction of the halogenated hydrocarbon with the reaction product of the carboxylic acid ester.

Compounds of this first group are represented by the formula (C):

(C) $R_fX$

(where X is as defined for (B), except that X does not represent a fluorine atom and $R_f$ is a fluorine containing aliphatic group having 1 to 20 carbon atoms).

Within this first group, an optional preferred feature is that X is bonded to a saturated carbon atom.

The compound containing the aliphatic group containing fluorine which is represented by $R_f$ in the general formula (C) of this type includes halogenated perfluoroalkyl compounds of straight or branched chain such as

$CF_3(CF_2)_pX$, $(CF_3)_2CF(CF_2)_pX$ and

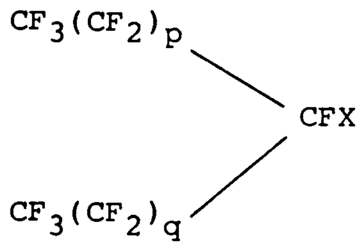

(where X represents a chlorine, bromine or an iodine atom, p and q are integers of zero or larger) and those compounds which contain hydrogen atom(s) in the molecular chain such as

$CF_3CH_2CH_2X$, $HCF_2(CF_2)_pX$ and $CF_3(CF_2)_pCH_2X$

(where X and p have the same meaning as before).

Further, $R_f$ may contain an unsaturated bond such as C = C double bond.

A preferred type of compound within formula (C) is a halogenated alkene containing fluorine expressed by the general formula,

$CH_2 = C(R_f)X$

In that case even a perfluoro compound may be locally substituted by a hydrogen atom. Such compounds include

$CF_2 = CF(CF_2)_pX$, $CF_3(CF_2)_pCF = CF(CF_2)_qX$, $CH_2 = CH(CF_2)_pX$

(where X, p and q have the same meaning as before).

Alternatively, within this first group, an optional preferred feature is that $R_f$ represents an unsaturated group having 2 to 20 carbon atoms, and X is bonded to an unsaturated carbon atom.

Further for $R_f$ of this type, even if the compound is a perfluoro compound, a part of $R_f$ may be substituted by hydrogen atom. Such compounds include,

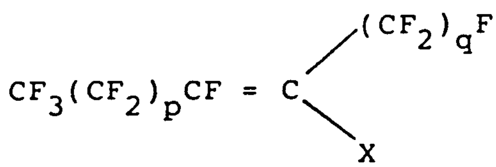

(where X, p and q have the same meaning as before),

$F_2C = CHX$, $HFC = CFX$, $HFC = CHX$, $H_2C = CFX$,
$F_2C = C(R)X$, $F(R)C = C(F)X$, $H(R)C = C(F)X$, $F(R)C = C(H)X$,
$H(F)C = C(R)X$, $H(R_f'')C = C(F)X$, $F(R_f'')C = C(H)X$, $H(F)C = C(R_f'')X$,
$H_2C = C(R_f'')X$, $R_2C = C(F)X$, $R_f''(R)C = C(F)X$, $R_f''_2C = C(H)X$,
$R_f''(R)C = C(H)X$, $F(R)C = C(R)X$, $R_f''(F)C = C(R_f'')X$ ,
$R_f''_2C = C(R_f'')X$, $R_f''(R)C = C(R)X$, $R_2C = C(R_f'')X$, $R_{f2}C = C(R)X$,
$R_f''(R)C = C(R_f'')X$, $R(F)C = C(R_f'')X$, $R_f''(F)C = C(R)X$,
$R_f''(H)C = C(R_f'')X$, $R_f''(H)C = C(R)X$, $R(H)C = C(R_f'')X$

(where R is an alkyl group, $R_f''$ is a fluorine containing aliphatic group and X has the same meaning as before).

Compounds listed below are exemplified as belonging to this group:

$CF_3I$, $CF_3Br$, $CF_3CF_2I$, $CF_3CF_2Br$, n-$C_3F_7I$,
$(CF_3)_2CFI$, n-$C_4F_9I$, n-$C_5F_{11}I$,
$(CF_3)_2CFCF_2CF_2I$, n-$C_6F_{13}I$, n-$C_7F_{15}I$,

$(CF_3)_2CF(CF_2CF_2)_2I$,
$n\text{-}C_8F_{17}I$, $n\text{-}C_{10}F_{21}I$, $n\text{-}C_{12}F_{25}I$, $n\text{-}C_{14}F_{29}I$,
$CF_2=CFCF_2I$, $CF_2=CFCF_2Br$,
$CF_3CH_2CH_2I$, $CF_3CH_2CH_2Br$,
$CH_2=CHCF_2Br$, $CH_2=CHCF_2CF_2Br$,
$CH_2=C(CF_3)Cl$, $CH_2=C(CF_3)Br$, $CH_2=C(CF_3)I$,
$CH_2=C(CF_2CF_3)Cl$, $CH_2=C(CF_2CF_3)Br$, $CH_2=C(CF_2CF_3)I$,
$CH_2=C[CF(CF_3)_2]Cl$, $CH_2=C[CF(CF_3)_2]Br$, $CH_2=C[CF(CF_3)_2]I$,
$CH_2=C(CF_2CF_2CF_2CF_3)Cl$, $CH_2=C(CF_2CF_2CF_2CF_3)Br$,
$CH_2=C(CF_2CF_2CF_2CF_3)I$,
$CH_2=C(CHF_2)Cl$, $CH_2=C(CHF_2)Br$, $CH_2=C(CHF_2)I$,
$CH_2=C(CH_2F)Cl$, $CH_2=C(CH_2F)Br$, $CH_2=C(CH_2F)I$,
$CH_2=C(CH_2CH_3)Cl$, $CH_2=C(CH_2CF_3)Br$, $CH_2=C(CH_2CF_3)I$,
$CF_2=CFBr$, $CF_2=CFI$, $HFC=CFI$, $H_2C=CFBr$, $H_2C=CFI$,
$CH_3CH_2CH(CH_3)CF=CFBr$, $CH_3CH_2CH(CH_3)CF=CFI$.

Preferred compounds among the listed are exemplified as follows:
a halogenated vinyl compound containing fluorine,

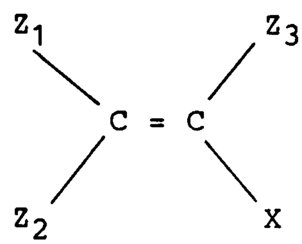

(where $Z_1$, $Z_2$ and $Z_3$ each independently represent either a hydrogen or a fluorine atom, but at least one among $Z_1$, $Z_2$ and $Z_3$ is a fluorine atom. X is a chlorine, bromine or iodine atom),
a perfluoroalkylene diiodide,

$C_nF_{2n+1}.I$

(where n is an integer between 1 and 20) and
an alpha-perfluoroalkyl substituted vinyl halogenide,

$H_2C=C(C_nF_{2n+1})X$

(where n is an integer between 1 and 18 and X is preferably a bromine or an iodine atom).

For example, following compounds are included:
perfluorooctyliodide, perfluorohexyliodide, perfluorobutyliodide, 3,3,3-trifluoro-2-bromopropene, 3,3,3-trifluoro-2-iodopropene, 3,3,4,4,5,5,6,6,6-nonafluoro-2-iodohexene, and 1,2,2-trifluoro-1-iodoethene.

Another preferred group of organic halogenated compounds containing fluorine is represented by the formula (B) in which neither X nor X′ represents a fluorine atom and $R_f$ is a fluorine containing bifunctional group. Within this group, preferably each of X and X′ is independently bonded to a saturated carbon atom.

A compound belonging to this group involves the reaction at the sites of X and X′ in the Reaction Step (I) and gives a dicarboxylic acid ester as a final product. Included in this group of compounds are, for example, perfluorodihalogen compounds of straight or branched chain expressed by general formulae,

$X(CF_2)_lX'$ or 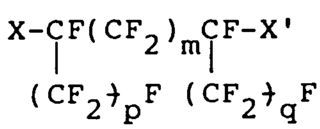

(where X and X′ are each independently chlorine, bromine or iodine atom. l and m are integers 3 or larger and 1 or larger, respectively, and p and q are integers 0 or larger)

and dihalogen compounds in which some fluorine atoms in the fluoroalkyl group are substituted by hydrogen atoms expressed by a general formula,

$X\text{-}CF_2(CF_2)_pCH_2CH_2(CF_2)_qCF_2\text{-}X'$

(where X, X′, p, and q have the same meaning as above).

Furthermore, $R_f$ or fluorine containing aliphatic group in the general formula (B) may be an unsaturated dihalogen group which contains unsaturation bonding such as C=C double bond. Following compounds may be exemplified as belonging to the mentioned type of compounds:

$X(CF_2CF_2)_pCF = CF(CF_2CF_2)_qX'$
$X\text{-}CF_2(CF_2)_pCH_2CF = CFCH_2(CF_2)_qCF_2\text{-}X'$

(where X, X′, p and q are the same as before).

Compounds belonging to this group may be exemplified as follows:

$I(CF_2)_3I$, $I(CF_2)_3Cl$, $Br(CF_2)_3Br$, $I(CF_2)_4I$,
$I(CF_2)_4Cl$, $Br(CF_2)_4Br$, $I(CF_3)CFCF_2CF_2I$,
$I(CF_3)CFCF_2CF_2Cl$, $Br(CF_3)CFCF_2CF_2Br$,
$ICF_2CF_2CF=CFCF_2CF_2I$, $ICF_2CF_2CF=CFCF_2CF_2Cl$,
$BrCF_2CF_2CF=CFCF_2CF_2Br$, $ICF_2CF_2CH_2CH_2I$,
$ICF_2CF_2CH_2CH_2Cl$ and $BrCF_2CF_2CH_2CH_2Br$.

Preferable compounds in this group, are perfluoroalkylenediiodides of straight or branched chain expressed by the following formula:

$I.C_nF_{2n}.I$

(where n is an integer 3 to 20), including, for example
α,ω-diiodoperfluorohexane and
α,ω-diiodoperfluorobutane.

Thus, a variety of compounds may be employed for the organic halogen compounds containing fluorine which can be expressed by the general formula (B). In practice, however, the number of carbon atoms composing said organic halogen compound containing fluorine is preferably 20 or less to maintain the practical solubility of the compounds in the solvent in which the reaction takes place.

In the reaction of the organic halogenated compound containing fluorine with carbon dioxide in the presence of zinc various solvents may be used, but the solvent in which the reaction takes place should be selected with consideration to a high yield of reaction. The preferable solvents in the method of the present invention are aprotic polar solvents, e.g. dimethylformamide (DMF), dimethylsulfoxide (DMSO), N,N-dimethylacetamide, tetramethyl urea, hexamethylphosphoramide, sulforane, N-methylpyrrolidone, 1,3-dimethyl-2-imidazolidinone, nitrobenzene, nitromethane, acetonitrile, propylene carbonate, tetrahydrofurane, dioxane, ether, diglyme, triglyme, and pyridine. From the viewpoint of yield, more preferable are DMF, 1,3-dimethyl-2-imidazolidinone, DMSO, N-methylpyrrolidone, N,N-dimethylacetamide, tetramethyl urea and hexamethylphosphoramide and especially DMF is the most preferable among them all.

Zinc used in the method of the present invention may be employed in the form of powder, the mean size of particles in diameter preferably ranging from 0.1 to 100 µm. In case that the particle is smaller than 0.1 µm in diameter, the procedure for removing it after the reaction will become complicated, and in case that it is larger than 100 µm in diameter, the yield of reaction will get smaller due to decrease in the effective area used during the reaction. Taking yield and procedure into consideration, the mean size of particles is preferably, in particular, 1 to 50 µm in diameter.

The amount of zinc can be decreased by pretreating the surface of zinc, though commercially available zinc powder can be utilized without treatment. The method for treatment of surface is carried out according to the method by Horben-Weyl (Horben-Weyl, 13(2a), pp.570 - 574 and p.815): pretreatment with acid treatment agents (mineral acids or acetic acid) and formation pairs with other metals, e.g., in general, copper, lead, cadmium, mercury, etc.

Zinc powder may be used in an amount ranging from 1 to 10 equivalents against the halogen atoms other than fluorine contained in the organic halogen compound containing fluorine as a starting material, but 2 to 10 equivalents are preferred to realize better reproducibility of the reaction.

In the method of the present invention, the reaction in this reaction step can also be carried out in the presence of a cation selected from ions of alkali metals, alkaline earth metals and ammonium.

In the present invention, an ammonium ion means $NH_4^+$ or one that all or a part of hydrogen atoms in $NH_4^+$ are substituted by at least one sort of substituents selected from alkyl, aryl, or alkylene, or a cation wherein a substituent selected from hydrogen, alkyl, or aryl combines to the nitrogen atom of pyridine.

Examples of such cations are as follows:

$Li^+$, $Na^+$, $K^+$, $Rb^+$, $Cs^+$, $Be^{2+}$, $Mg^{2+}$, $Ca^{2+}$, $Sr^{2+}$, $Ba^{2+}$, $NH_4^+$, $N(CH_3)_4^+$, $N(C_2H_5)_4^+$, $N(n\text{-}C_3H_7)_4^+$, $N(n\text{-}C_4H_9)_4^+$, $NH(CH_3)_3^+$, $NH(C_2H_5)_3^+$, $NH(n\text{-}C_3H_7)_3^+$, $NH(n\text{-}C_4H_9)_3^+$,

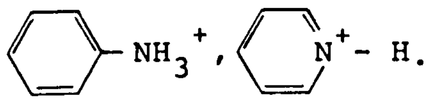

These cations can be applied to the present invention individually or in combination of several sorts. Furthermore, these cations can be easily supplied by addition of compounds consisting of the cations of concern and anions to the reaction system.

Said anions, though not restricted especially, are exemplified as follows:

$F^-$, $Cl^-$, $Br^-$, $I^-$, $OH^-$, $O_2^-$, $ClO_2^-$, $ClO_3^-$, $ClO_4^-$,
$BF_4^-$, $S_2O_3^{2-}$, $SO_4^{2-}$, $NO_2^-$, $NO_3^-$, $N_3^-$, $PO_4^{3-}$,
$CH_3O^-$, $C_2H_5O^-$, $CH_3S^-$, $CN^-$, $SCN^-$,
$CO_3^{2-}$, $HCO_3^-$, $CH_3COO^-$, and $C_2O_4^{2-}$.

The amount of coexisting cations is preferably within the range from 0.01 to 50 gram atoms against 1 mole of the halogen atoms other than fluorine in the organic halogen compound containing fluorine employed in the method and represented in the general formula (B). An amount of cation less than 0.01 gram atom hardly exhibits the effect of promoting yield and an amount exceeding 50 gram atoms does not allow the yield to be promoted to an extent comparable to the amount.

The reaction in this reaction step may be carried out within a wide extent of temperature, but preferably in a range between 0 and 150 ° C in an ordinary case. A temperature below 0 ° C is not practical since a long time is required for the reaction to promote conversion of the raw material. A temperature above 150 ° C extremely lowers the selectivity to carboxylic acid esters which is achieved after the Reaction Step (II), because of increase in the ratio of side reactions.

The reaction of an organic halogen compound containing fluorine is carried out in an organic solvent under suspension of zinc alone or together with cations referred to above by the contact with carbon dioxide at a certain temperature. Various methods can be employed for the reaction. For example,

(1) a method of adding the organic halogen compound containing fluorine to a solution in organic solvent in which powders of zinc alone or together with other metals, or zinc and the cations described above, are suspended while carbon dioxide gas is bubbled under a normal pressure,

(2) the method in (1) which is carried out while the reaction system is being irradiated by ultrasonic waves, and

(3) the method in (1) which is carried out under a pressure of carbon dioxide using an autoclave.

However, there is not any restriction with the present invention.

In addition, carbon dioxide may be used in an amount not less than equimolar to that of the halogen atoms other than fluorine in the fluorine containing organic halogen compound as starting material, and the reaction time, from a half to 10 hours, suffices in this reaction step after certain reaction conditions being settled.

The reaction mixture obtained in the reaction of the organic halogenated compound containing fluorine with carbon dioxide in the presence of zinc is then transferred to the second stage of the process.

In cases when zinc is used in the reaction of the organic halogenated compound containing fluorine with carbon dioxide in the presence of zinc in an amount excessive to that of halogen atoms other than fluorine in the fluorine containing organic halogen compound as starting material and powders of zinc alone or together with those of other metals and salts of cations employed are contaminated in a solid form in the reaction mixture, the remaining metal powders and salts of cations employed are preferably removed in the reaction of the organic halogenated compound containing fluorine with carbon dioxide in the presence of

zinc by an operation such as filtration prior to the reaction of the second stage of the process.

The halogenated hydrocarbon employed in this reaction step which is expressed by the general formula (A) encompasses a variety of compounds

(A) RY

R represents a straight or branched chained alkyl group having 1 to 20 carbon atoms which optionally contains fluorine.

a saturated or unsaturated, straight or branched chained aliphatic group having up to 20 carbon atoms which is substituted by an aromatic ring,

or an alkenyl group expressed by a general formula,

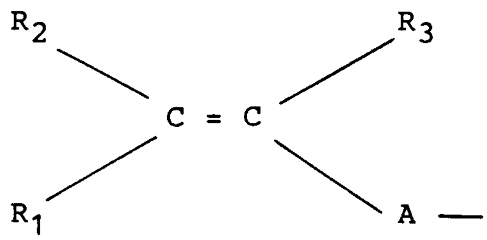

(where $R_1$, $R_2$ and $R_3$ independently represent a hydrogen atom or a straight or branched chained alkyl group and A represents a methylene group or a straight or branched chained alkylene group having 3 to 20 carbon atoms)

and Y represents a chlorine atome, a bromine atom or an iodine atom.

Preferably, R is a saturated straight or branched chained group having 1 to 20 carbon atoms, which is more preferably a fluorine containing saturated alkyl group.

Examples of alkyl halides having a saturated alkyl group of straight or branched chain which are expressed by general formulae,

$CH_3(CH_2)_nY$ and $(CH_3)_2CH(CH_2)_nY$

(where n is an integar, 0 or larger, Y is any of chlorine, bromine and iodine atoms). They are exemplified by the following compounds:

$CH_3I$, $CH_3Br$, $CH_3Cl$, $CH_3CH_2I$, $CH_3CH_2Br$, $CH_3CH_2Cl$,
n-$C_3H_7I$, n-$C_3H_7 \bullet Br$, iso-$C_3H_7I$, iso-$C_3H_7Br$, iso-$C_3H_7Cl$,
n-$C_4H_9I$, n-$C_4H_9Br$, $(CH_3)_2CH \bullet CH_2I$, $(CH_3)_2CH \bullet CH_2Br$,
n-$C_5H_{11}I$, n-$C_5H_{11}Br$, n-$C_5H_{11}Cl$,
$(CH_3)_2CHCH_2CH_2I$, $(CH_3)_2CHCH_2CH_2Br$.

Another preferred group are the alkenyl halides having an unsaturated alkenyl group of straight or branched chain expressed by general formula,

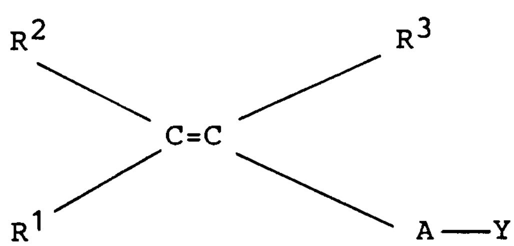

including $CH_2=CH(CH_2)_nY$, $H(CH_2)_nCH=CH(CH_2)_mY$ and $(CH_3)_2CHCH=CH(CH_2)_nY$
(where n and m are integers 1 or larger and Y has the same meaning as above). Following are examples

$CH_2=CHCH_2Cl$, $CH_2=CHCH_2Br$, $CH_2=CHCH_2I$, $CH_3CH=CHCH_2Cl$,

$CH_3CH=CHCH_2Br$, $CH_3CH=CHCH_2I$, $CH_2=CHCH_2CH_2Cl$,
$CH_2=CHCH_2CH_2Br$, $CH_2=CHCH_2CH_2I$, $CH_2=CHCH(CH_3)Cl$,
$CH_2=CHCH(CH_3)Br$, $CH_2=CHCH(CH_3)I$, $CH_2=C(CH_3)CH_2Cl$,
$CH_2=C(CH_3)CH_2Br$, $CH_2=C(CH_3)CH_2I$, $C_2H_5CH=CHCH_2Cl$,
$C_2H_5CH=CHCH_2Br$, $C_2C_5CH=CHCH_2I$, $CH_3CH=CHCH_2CH_2Cl$,
$CH_3CH=CHCH_2CH_2Br$, $CH_3CH=CHCH_2CH_2I$, $CH_2=C(CH_3)CH_2CH_2Cl$,
$CH_2=C(CH_3)CH_2CH_2Br$, $CH_2=C(CH_3)CH_2CH_2I$, $(CH_3)_2C=CHCH_2Cl$,
$(CH_3)_2C=CHCH_2Br$, $(CH_3)_2C=CHCH_2I$, $(CH_3)_2C=C(CH_3)CH_2Cl$,
$(CH_3)_2C=C(CH_3)CH_2Br$, $(CH_3)_2C=C(CH_3)CH_2I$.

The halogenated hydrocarbon may be substituted by an aromatic ring, so that R is a saturated or unsaturated straight or branched chained aliphatic group of up to 20 carbon atoms, substituted by an aromatic ring.

Examples include alkyl halides having an alkyl group of straight or branched chain which is substituted by aromatic ring represented as benzene substituted alkyl halides expressed by general formulae,

$C_6H_5(CH_2)_nY$ and $C_6H_5CH[(CH_2)_nCH_3]Y$,

(where n is an integer being 1 or larger and Y has the same meaning as above), such as

$C_6H_5CH_2Cl$, $C_6H_5CH_2Br$, $C_6H_5CH_2I$,
$C_6H_5CH(CH_3)Cl$, $C_6H_5CH(CH_3)Br$, $C_6H_5CH(CH_3)I$,
$C_6H_5CH_2CH_2Cl$, $C_6H_5CH_2CH_2Br$, $C_6H_5CH_2CH_2I$,
$C_6H_5CH(CH_2CH_3)CH_2Cl$, $C_6H_5CH(CH_2CH_3)CH_2Br$,
$C_6H_5CH(CH_2CH_3)CH_2I$, $C_6H_5C(CH_3)_2Cl$, $C_6H_5C(CH_3)_2Br$,
$C_6H_5C(CH_3)_2I$, $C_6H_5CH_2CH(CH_3)Cl$, $C_6H_5CH_2CH(CH_3)Br$,
$C_6H_5CH_2CH(CH_3)I$, $C_6H_5CH_2CH_2CH_2Cl$, $C_6H_5CH_2CH_2CH_2Br$,
$C_6H_5CH_2CH_2CH_2I$.

In addition, those halogen containing unsaturated hydrocarbons having an alkenyl group of straight or branched chain which contains a C=C double bond and is substituted by an aromatic ring may be also employed. Those compounds are expressed by the following formulae,

$C_6H_5(CH=CH)(CH_2)_nX$ and $C_6H_5CH(CH_3)\text{-}CH=CH(CH_2)_nX$

(where n and X have the same meaning as before). These compounds can be exemplified concretely by the following compounds:

$C_6H_5CH=CH\text{-}CH_2Cl$, $C_6H_5CH=CH\text{-}CH_2Br$, $C_6H_5CH=CH\text{-}CH_2I$,
$C_6H_5CH=CH\text{-}CH_2CH_2Cl$, $C_6H_5CH=CH\text{-}CH_2CH_2Br$,
$C_6H_5CH=CH\text{-}CH_2CH_2I$,
$C_6H_5CH=CH\text{-}CH(CH_3)Cl$, $C_6H_5CH=CH\text{-}CH(CH_3)Br$,
$C_6H_5CH=CH\text{-}CH(CH_3)I$,
$C_6H_5CH_2CH=CHCH_2Cl$, $C_6H_5CH_2CH=CHCH_2Br$, $C_6H_5CH_2CH=CHCH_2I$,
$C_6H_5CH(CH_3)\text{-}CH=CHCH_2CH_2Cl$, $C_6H_5CH(CH_3)\text{-}CH=CHCH_2CH_2Br$,
$C_6H_5CH(CH_3)\text{-}CH=CHCH_2CH_2I$.

Furthermore, even if the aromatic ring which substitutes a hydrogen atom on an alkyl halogen compound has any substituent group, the alkyl halogen compound containing an aromatic ring may be employed as long as the substituent group does not interfere with the esterification reaction. Following compounds are examples thereof:

$CH_3\text{-}C_6H_4CH_2Cl$, $CH_3OC_6H_4CH_2Cl$, $NC\text{-}C_6H_4CH_2Cl$,
$O_2N\text{-}C_6H_4CH_2Cl$, $CF_3\text{-}C_6H_4\text{-}CH_2Cl$, $CH_3CH_2C_6H_4CH_2Br$,
$CH_3C_6H_4H_2I$, $(CH_3)_2C_6H_3CH_2Cl$, $(CH_3)_3C_6H_2CH_2Cl$,
$CH_3\text{-}C_6H_4CH_2Cl$, $CH_3\text{-}C_6H_4CH_2CH_2Br$, $CH_3\text{-}C_6H_4CH_2CH_2I$.

Further examples of alkyl groups of straight or branched chain containing fluorine are as follows:

$CF_3CH_2Cl$, $CF_3CH_2Br$, $CF_3CH_2I$,
$CF_3CF_2CF_2CF_2CH_2CH_2Br$, $CF_3CF_2CF_2CF_2CH_2CH_2I$,
$(CF_3)_2CFCH_2CH_2Br$, $(CF_3)_2CFCH_2CH_2I$,
$(CF_3)_2CFCF_2CF_2CH_2CH_2Br$, $(CF_3)_2CFCF_2CF_2CH_2CH_2I$,
n-$C_6F_{13}CH_2CH_2Br$, n-$C_6F_{13}CH_2CH_2I$,
n-$C_8F_{17}CH_2CH_2Br$, n-$C_8F_{17}CH_2CH_2I$.

Following compounds are considered as preferable among the said halogenated hydrocarbons: ethylbromide, methyliodide, n-propyliodide, n-butyliodide, 1-iodo-3-methylbutane, allylchloride, allylbromide, 3-chloro-1-butene, benzylchloride, benzylbromide, 3,3,4,4,5,5,6,6,7,7,8,8,8-tridecafluoro-1-iodooctane, and 3,3,4,4,5,5,6,6,7,7,8,8,9,9,10,10,10-heptadecafluoro-1-iodododecane.

A variety of compounds may be employed as the halogenated hydrocarbon expressed by the general formula (A). However, the practical solubility of these compounds in the solvent in which the reaction is carried out can be maintained preferably by employing those halogenated hydrocarbons which contain 20 or less carbon atoms in total.

As for the RY employed, 1 to 20 times the molar quantity as that of the halogen atoms other than fluorine contained in the fluorine containing organic halogen compound of concern suffices for the practical use. If the amount of RY employed is less than equimolar to the halogen atoms other than fluorine contained in the fluorine containing organic halogen compound, the ester is formed only with a remarkably low yield. On the other hand, substantially no increase in yield is observed even if a more than 20 times as much amount in molar is used.

Solvents employed in this reaction step may be the same as those used in the reaction of the organic halogenated compound containing fluorine with carbon dioxide in the presence of zinc. Therefore, the resultant reaction mixture in the reaction of the organic halogenated compound containing fluorine with carbon dioxide in the presence of zinc from which powders of metal are removed if necessary may be used without any further treatment. The solvent replacement procedure may be applied to remove the solvent used in the reaction of the organic halogenated compound containing fluorine with carbon dioxide in the presence of zinc and to introduce a new solvent for convenience in the operation and to improve the yield of reaction. In such a case aprotic solvents are preferred.

Preferable aprotic solvents are exemplified as follows:

DMF, DMSO, N,N-dimethylacetamide, tetramethyl urea, hexamethylphosphoramide, sulforane, N-methylpyrrolidone, 1,3-dimethyl-2-imidazolidinone, nitrobenzene, nitromethane, acetonitrile, propylene carbonate, tetrahydrofurane, dioxane, ether, diglyme, triglyme, and pyridine.

In this reaction step, the reaction mixture obtained by the reaction of the organic halogenated compound containing fluorine with carbon dioxide in the presence of zinc is reacted with a halogenated hydrocarbon at a certain temperature for a certain predetermined time while the whole mixture being mixed and agitated. The reaction can be carried out in a wide range of temperature 0° to 300°C. If the reaction occurs at a temperature below 0°C, a longer time is required before the reaction is completed, while at a temperature of reaction above 300°C by-products are formed in a larger proportion and the yield tends to decrease. The time of reaction varies to a large extent depending on the temperature of reaction, but it may be set in a range from 10 min. to 500 hr.

From the reaction mixture containing the carboxylic acid ester having fluorine which is obtained in the process described above, the carboxylic acid ester containing fluorine can be isolated employing various methods. For example, when the solvent used is an aqueous one, the reaction mixture is transferred to water or aqueous acid solution and an isolated organic layer is rectified. Otherwise, the reaction mixture is simply distilled for rectification.

Using the process of this invention, carboxylic acid esters containing fluorine can be prepared in very simple and convenient operations from organic halogen compounds containing fluorine with a high yield. Therefore, the present invention permits carboxylic acid esters containing fluorine which are a important intermediate for synthesis in industry to be produced at a lower price and in high purity, promising a large merit in industry.

A better understanding of the present invention and its many advantages will be had by referring to the following specific examples, given by way of illustration.

Example 1

Reaction Step (I)

In a 200 cc autoclave of the electromagnetic agitation type provided with two openings for introduction of carbon dioxide and pumping under pressure of perfluoroalkyliodide, 25.6 g (0.385 gram-atom) of zinc powder (mean diameter of particles of about 15 µm) which was washed beforehand with a 0.5 N aqueous hydrochloric acid solution and dried was placed and the inside of the autoclave was warmed to 15° C by the heating from outside. The pressure of carbon dioxide was set to 8.0 kg/cm$^2$ (read-out on guage) by means of a constant pressure device so that this pressure of the carbon dioxide in the inside of the autoclave was maintained to the end of reaction. Then, while the inside of the autoclave being agitated, 100 ml of DMF was added with a liquid delivery pump.

Subsequently, a mixture of 66.1 g (or 0.121 mol) of perfluorooctyliodide and 16 ml of DMF was introduced under pressure into the autoclave at a constant rate for 2 hr, using a liquid delivery pump.

Stirring was continued at the same temperature for an additional 2 hr, then the pressure of carbon dioxide in the autoclave was returned to the normal atmospheric pressure to terminate the reaction.

Reaction Step (II)

The remaining zinc powders were removed by filtration from the reaction mixture. To the said reaction mixture, after removal of zinc, 38,4 g (or 0.353 mol) of ethylbromide was added and mixed, and the mixture was placed again in the autoclave. Reaction was carried out at 110° C under stirring for 5 hr.

The reaction mixture thus obtained was poured in an aqueous 1N hydrochloric acid solution and the isolated organic layer was rectified under a reduced pressure, to obtain 53.6 g (or 0.109 mol) of perfluorononanoic acid ethyl ester.

Yield : 90.1 %
Boiling point: 82.0 - 83.5° C/18 mm Hg.

Example 2

The Reaction Step (I) was conducted in the same way as in Example 1.

Zinc powder remaining in the reaction mixture was removed by filtration. To the reaction mixture from which zinc had been removed 50.1 g (or 353 mol) of methyiodide was added and mixed by agitation for 75 hr. at the room temperature.

Reaction mixture thus obtained was poured into a 1N hydrochloric acid solution and a organic layer isolated was rectified under a reduced pressure, to obtain 53.2 g (or 0.111 mol) of perfluorononanoic acid methyl ester.

Yield : 91.7 %,
Boiling point: 78.2 - 79.5° C/21 mm Hg.

Example 3

Reaction Step (I)

The Reaction Step (I) was conducted in the same manner as in the Reaction Step (I) in Example 1, except that 54.0 g (or 0.121 mol) of perfluorohexyliodide was employed in place of perfluorooctyliodide.

Reaction Step (II)

Remaining zinc powder was removed by filtration from the reaction mixture. With the said reaction mixture from which zinc had been removed, 50.6 g (or 0.356 mol) of methyliodide was mixed, the mixed solution was placed in the autoclave and the reaction was carried out at 80° C under stirring for 7 hr. Reaction mixture thus prepared was poured into a 1N hydrochloric acid solution, an organic layer isolated was rectified under a reduced pressure, to obtain 42.1 g (or 0.111 mol) of perfluoroheptanoic acid methyl ester.

Yield : 91.7 %,
Boiling point: 67 - 68° C/55 mm Hg.

Example 4

Reaction Step (I)

The same procedure as that in the Reaction Step (I) in Example 1 was followed except that 41.9 g (or 0.121 mol) of perfluorobutyliodide was employed in place of perfluorooctyl iodide.

Reaction Step (II)

Remaining zinc powder in the reaction mixture was removed by filtration. To the reaction mixture from which zinc had been removed, 51.0 g (or 0.359 mol) of methyliodide was added and the whole was mixed. Then the mixture was placed in the autoclave and allowed to react for 7 hr. under stirring at 80 ° C.

Reaction mixture thus prepared was poured into an aqueous 1N hydrochloric acid solution and an organic layer isolated was rectified, to obtain 30.4 g (or 0.109 mol) of perfluoroheptanoic acid methyl ester.

Yield : 90.1 %,
Boiling point: 93.5 - 94.0 ° C.

Examples 5 - 7

The same procedure as in Example 2 was followed except that 0.356 mol of alkyliodide compounds shown in Table 1 were employed in place of methyliodide and the reaction was carried out at 90 ° C for 8 hr.

The products obtained and the yield are shown in Table 1.

Table 1

| Example | Alkyliodide | Product | Yield (%) |
|---|---|---|---|
| 5 | $n-C_3H_7I$ | $n-C_8F_{17}COOC_3H_7$ | 92.3 |
| 6 | $n-C_4H_9I$ | $n-C_8H_{17}COOC_4H_9$ | 90.2 |
| 7 | $(CH_3)_2CHCH_2CH_2I$ | $n-C_8F_{17}COOCH_2CH_2CH(CH_3)_2$ | 88.7 |

Examples 8 - 10

The same procedure as in Example 1 was followed except that the solvents indicated in Table 2 were employed in place of DMF. The whole result is shown in Table 2.

Table 2

| Example | Solvent | Yield (%) |
|---|---|---|
| 8 | N-methylpyrrolidone | 84.2 |
| 9 | N,N-dimethylacetamide | 86.7 |
| 10 | Dimethylsulfoxide | 80.5 |

Example 11

Reaction Step (I)

In a 200 cc autoclave of electromagnetic agitation type which is provided with two pressure inlets, one for carbon dioxide and the other for perfluoroalkyliodide, was placed 25.1 g (or 0.383 gram-atom) of zinc powder (with a mean particle diameter about 15 µm) which was washed beforehand with a 0.5 N aqueous hydrochloric acid solution and the temperature inside the autoclave was made 15 °C by heating from outside. The pressure of carbon dioxide was made to 8.0 $kg/cm^2$ (reading on guage) by means of a constant pressure device and this value of pressure was maintained to the completion of reaction. Then 100 ml of DMF was introduced in the autoclave with a liquid delivery pump under stirring.

Subsequently, a mixture of 65.0 g (or 0.119 mol) of perfluorooctyliodide and 16 ml of DMF was introduced under pressure into the autoclave at a constant speed in 2 hr. Agitation was continued for 2 additional hours at the same temperature, then the pressure of carbon dioxide inside the autoclave was reduced to the atmospheric pressure, to terminate the reaction.

Reaction Step (II)

The remaining zinc powder was removed by filtration from the reaction mixture. The reaction mixture was mixed with 30.9 g (or 0.404 mol) of allylchloride and the whole mixture was again placed in the autoclave and allowed to react at 110 °C for 3 hr.

Reaction mixture thus obtained was poured in an aqueous 2N hydrochloric acid solution and an organic layer isolated was rectified under a reduced pressure, obtain 39.9 g (or 0.0791 mol) of perfluorononanoic acid allylester.

Yield : 66.5 %,
Boiling point: 84. °C/10.5 mm Hg.

Example 12

The Reaction Step (I) was carried out in the same manner as in Example 11. Then remaining zinc powder was removed by filtration from the reaction mixture. To this reaction mixture was mixed 44.4 g (or 0.367 mol) of allylbromide and allowed to react under stirring for 3 hr. at 110 °C.

Reaction mixture thus obtained was poured in an aqueous 2N hydrochloric acid solution and an organic layer isolated was separated and rectified under a reduced pressure, to obtain 47.4 g (or 0.0940 mol) of perfluorononanoic acid allylester.

Yield: 79 %.

Example 13

Reaction Step (I)

The same procedure as that of the Reaction Step (I) in Example 11 was followed except that 41.2 g (or 0.119 mol) of perfluorobutyliodide was employed in place of perfluorooctyliodide.

Reaction Step (II)

From the reaction mixture obtained in the Reaction Step (I) above remaining zinc powder was removed by filtration and the rest of the mixture was reacted with allylchloride in the same manner as in the Reaction Step (II) in Example 11. Reaction mixture obtained was poured in an aqueous 2N hydrochloric acid solution and an organic layer isolated was rectified under a reduced pressure, to obtain 24.7 g (or 0.0813 mol) of perfluoropentanoic acid allylester.

Yield: 68.3 %.

Example 14

The same reaction as that in the Reaction Step (I) of Example 11 was carried out and the zinc powder remaining in the reaction mixture was removed by filtration. To this reaction mixture was further mixed with 35.9 g (or 0.396 mol) of 3-chloro-1-butene and placed again in the autoclave. The whole mixture was allowed to react at 110 °C for 5 hr. while being agitated. Reaction mixture obtained was poured in an aqueous 2N hydrochloric acid solution and an organic layer isolated was rectified under a reduced pressure, to obtain 40.1 g (or 0.0774 mol) of 1-methylallyl ester of perfluorononanoic acid.

Yield: 65.0 %

Example 15

The same reaction was followed as that in the Reaction Step (I) in Example 11. Reaction mixture obtained was filtered to remove remaining zinc powder. To the reaction mixture was added 48.5 g (or 0.359 mol) of 4-bromo-1-butene and mixed together. The mixture was placed again in the autoclave and reaction was carried out at 110 ° C for 3 hr. under stirring. Reaction mixture thus obtained was poured in an aqueous 2N hydrochloric acid solution and an organic layer isolated was rectified under a reduced pressure, to obtain 47.6 g (or 0.0918 mol) of 4-butenyl ester of perfluorononanoic acid.

Yield: 77.1 %.

Examples 16 - 18

Experiment was carried out entirely in the same procedure as that in Example 11 except that those solvents shown in Table 3 were employed in place of DMF. Results are put together and shown in Table 3.

Table 3

| Example | Solvent | Yield (%) |
|---|---|---|
| 16 | N-methylpyrrolidone | 62.1 |
| 17 | N,N-dimethylacetamide | 64.0 |
| 18 | Dimethylsulfoxide | 59.4 |

Example 19

Reaction Step (I)

In a 200 cc autoclave of electromagnetic agitation type provided with two pressure inlets, one for carbon dioxide and the other for perfluoroalkyliodide, was placed 39.2 g (or 0.60 gram-atom) of zinc powder (having mean particle diameter of about 15 µm) which had been washed beforehand with a 0.5 N aqueous hydrochloric acid solution and dried. The inside of the autoclave was made 5 ° C by heating from outside. Carbon dioxide was introduced up to a pressure of 8.5 $kg/cm^2$ (reading on guage) by means of a constant pressure device. The pressure of carbon dioxide inside the autoclave was maintained to the completion of the reaction. Then 67 ml of DMF was introduced under agitation in the autoclave with a liquid delivery pump.

Subsequently a mixture consisting of 44.6 g (or 0.100 mol) of 1,4-diiodoperfluorobutane and 21 ml of DMF was introduced under pressure in the autoclave at a constant speed in 1.3 hr. The mixture was stirred for additional 4 hr. at the same temperature and then the pressure of carbon dioxide in the autoclave was released to the atomspheric pressure to terminate the reaction.

Reaction Step (II)

Zinc powder remaining in the reaction mixture was removed by filtration. This reaction mixture was further mixed with 60.2 g (or 0.552 mol) of ethylbromide and the whole was placed in the autoclave and allowed to react at 110 ° C for 5 hr.

The reaction mixture thus obtained was poured in a 1N aqueous hydrochloric acid solution and an isolated organic layer was rectified under a reduced pressure, to obtain 30.6 g (or 0.0883 mol) of perfluoroadipic acid diethyl ester.

Yield : 88.3 %,
Boiling point: 60.2 - 63.3 ° C/7 mm Hg.

Example 20

The Reaction Step (I) in Example 19 was carried out in the same manner as above and zinc powder remaining in the reaction mixture was removed by filtration. To this reaction mixture was mixed 76.4 g (or 0.538 mol) of methyliodide and agitated for mixing in the room temperature for 180 hr.

Reaction mixture thus obtained was poured in a 1N aqueous hydrochloric acid solution and an organic layer isolated was rectified under a reduced pressure, to obtain 27.9 g (or 0.0876 mol) of dimethyl ester of perfluoroadipic acid.

Yield : 68.6 %,
Boiling point: 71.5 - 73.5 °C/10 mm Hg.

Example 21

Reaction Step (I)

The same procedure of the Reaction Step (I) was followed as in Example 19 except that 55.4 g (or 0.100 mol) of 1,6-diiodoperfluorohexane was employed in place of 1,4-diiodoperfluorobutane which, as a mixture with 16 ml of DMF, was introduced into the autoclave with a pressure applied.

Reaction Step (II)

The reaction mixture was filtered to remove remaining zinc powder and mixed with 55.6 g (or 0.510 mol) of ethylbromide. The mixture was placed in the autoclave and allowed to react at 80 °C while being stirred for 10 hr. Reaction mixture thus obtained was poured in a 1N aqueous hydrochloric acid solution and an organic layer isolated was rectified under a reduced pressure, to obtain 39.0 g (or 0.0875 mol) of diethyl ester of perfluorosuberic acid.

Yield : 87.5 %,
Boiling point: 91 - 93.5 °C/4 mm Hg.

Examples 22 - 25

The same procedure as that in Example 20 was followed except that alkyliodide compounds shown in Table 4 was used in an amount of 0.531 mol in place of methyliodide and the reaction was carried out at 95 °C for 8 hr.

Products obtained and the corresponding yields are shown in Table 4.

Table 4

| Example | Alkyl or Alkenyl Iodide | Product | Yield (%) |
|---|---|---|---|
| 22 | n-$C_3H_7I$ | $H_7C_3OOC(CF_2)_4COOC_3H_7$ | 82.9 |
| 23 | n-$C_4H_9I$ | $H_9C_4OOC(CF_2)_4COOC_4H_9$ | 80.3 |
| 24 | $(CH_3)_2CHCH_2CH_2I$ | $(CH_3)_2CHCH_2CH_2OOC(CF_2)_4COOCH_2CH_2CH(CH_3)_2$ | 79.1 |
| 25 | $CH_2{=}CHCH_2Cl$ | $CH_2{=}CHCH_2OOC(CF_2)_4COOCH_2CH{=}CH_2$ | 62.3 |

Examples 26 - 28

The same procedure as that of Example 19 was followed except that the solvents shown in Table 5 were employed in place of DMF.

Results are shown in Table 5.

Table 5

| Example | Solvent | Yield (%) |
|---|---|---|
| 26 | N-methylpyrrolidone | 82.3 |
| 27 | N,N-dimethylacetamide | 83.7 |
| 28 | Dimethylsulfoxide | 79.8 |

Example 29

Reaction Step (I)

In a 200 ml autoclave of electromagnetic agitation type provided with two pressure inlets, one for carbon dioxide and the other for perfluoroalkyliodide, was placed 19.6 g (or 0.3 gram-atom) of zinc powder (having mean particle diameter of about 15 μm) which had been washed beforehand with 0.5 N hydrochloric acid and dried. The inside of the autoclave was made 15 °C by heating from outside. Carbon dioxide was introduced up to a pressure of 8.0 kg/cm$^2$ (reading on guage) by means of a constant pressure device. The pressure of carbon dioxide inside the autoclave was maintained to the completion of the reaction. Then 100 ml of DMF was introduced under agitation in the autoclave with a liquid delivery pump.

Subsequently a mixture consisting of 54.6 g (or 0.1 mol) of perfluorooctyliodide and 16 ml of DMF was introduced under pressure in the autoclave at a constant speed in 2 hr. The mixture was stirred for additional 2 hr. at the same temperature and then the pressure of carbon dioxide in the autoclave was released to the atmospheric pressure to terminate the reaction.

Reaction Step (II)

The reaction mixture obtained in the Reaction Step (I) above from which remaining zinc powder was removed by filtration was placed in a 3-necked round bottom flask of 300 ml capacity and 38 g (or 0.3 mol) of benzylchloride was added to it, which was allowed to react under stirring for 3 hr. at a temperature of 110 °C. After being cooled to the room temperature, the reaction solution was poured in a 6N hydrochloric acid solution, followed by extraction with diethyl ether and removal of the solvent DMF. The diethyl ether solution was dried with anhydrous magnesium sulfate and diethyl ether and benzylchloride were distilled off, to obtain 41.5 g (or 0.075 mol) of perfluorononanoic acid benzyl ester. Yield was 75 % based on perfluorooctyliodide.

Example 30

Reaction Step (I)

Excepting that 55.4 g (or 0.1 mol) of perfluorohexane-1,6-diiodide was employed in place of perfluorooctyliodide, the same procedure as that of Reaction Step (I) in Example 29 was followed.

Reaction Step (II)

The reaction mixture of the Reaction Step (I) from which remaining zinc powder had been removed by filtration was placed to a 3-necked round bottom flask of 500 ml capacity provided with a reflux condenser. Benzylchloride in an amount of 102.6 g (or 0.6 mol) was added and the mixture was allowed to react at 110 °C for 3 hr. while being stirred. After being cooled to the room temperature, the solvent DMF and

excess of benzylchloride were recovered by distillation under a reduced pressure, to obtain 38.5 g (or 0.0675 mol) of perfluorosuberic acid dibenzyl ester. A yield of 67.5 % was obtained.

Example 31

Reaction Step (I)

In a 200 cc autoclave of electromagnetic agitation type provided with two pressure inlets, one for carbon dioxide and the other for 3,3,3-trifluoro-2-iodopropene, was placed 19.6 g (or 0.3 gram-atom) of zinc powder (having mean particle diameter of about 15 µm) which had been washed beforehand with 0.5 N hydrochloric acid and dried . The inside of the autoclave was made 35 °C by heating from outside. Carbon dioxide was introduced up to a pressure of 6.0 kg/cm$^2$ (absolute pressure) by means of a constant pressure device. The pressure of carbon dioxide inside the autoclave was maintained to the completion of the reaction. Then 80 ml of DMF was introduced under agitation in the autoclave with a liquid delivery pump. Subsequently a mixture consisting of 22.2 g (or 0.10 mol) of 3,3,3-trifluoro-2-iodopropene and 10 ml of DMF was introduced under pressure in the autoclave at a constant speed in 1 hr. The mixture was stirred for additional 2 hr. at the same temperature and then the pressure of carbon dioxide in the autoclave was released to the atmospheric pressure to terminate the reaction.

Reaction Step (II)

The reaction mixture obtained in the Reaction Step (I) above from which remaining zinc powder was removed by filtration was mixed with 42.6 g (or 0.3 mol) of methyliodide and the resulting mixture was placed again in the autoclave. This was allowed to react under stirring for 3 hr. at a temperature of 110 °C. After the solution being cooled to the room temperature, toluene was added to the solution as internal standard to estimate the yield. The gas chromatographic determination demonstrated that the yield of 2-trifluoromethylacrylic acid methyl ester was 79 % based on 3,3,3-trifluoro-2-iodopropene as standard. From the reaction solution, the methyliodide employed in excess was recovered by distillation and subsequent distillation under a reduced pressure gave 10.0 g (or 0.065 mol) of 2-trifluoromethylacrylic acid methyl ester.

Example 32

Reaction Step (I)

In a 200 cc autoclave of electromagnetic agitation type provided with two pressure inlets, one for carbon dioxide and the other for 3,3,3-trifluoro-2-bromopropene, was placed 7.85 g (or 0.12 gram-atom) of zinc powder (having mean particle diameter of about 15 µm) which had been washed beforehand with 0.2 N hydrochloric acid and dried and 2.55 g (or 0.06 mol) of lithium chloride. The inside of the autoclave was made 35 °C by heating from outside. Carbon dioxide was introduced up to a pressure of 6.0 kg/cm$^2$ (absolute pressure) by means of a constant pressure device. The pressure of the carbon dioxide inside the autoclave was maintained to the completion of the reaction. Then 80 ml of DMF was introduced under agitation in the autoclave with a liquid delivery pump. Subsequently a mixture consisting of 7.0 g (or 0.04 mol) of 3,3,3-trifluoro-2-bromopropene and 26 ml of DMF was introduced under pressure in the autoclave at a constant speed in about 10 min. further 10 ml of DMF was introduced under pressure to clean the inside of the piping. The mixture was stirred for additional 4 hr. at the same temperature and then the pressure in the autoclave was released to the atmospheric pressure to terminate the reaction.

Reaction Step (II)

The reaction mixture obtained in the Reaction Step (I) above from which remaining zinc powder was removed by filtration was mixed with 15.2 g (or 0.12 mol) of benzyl chloride and the resulting mixture was placed again in the autoclave.

The mixture in the autoclave was allowed to react under stirring for 3 hr. at a temperature of 110 °C. After the solution being cooled to the room temperature, n-hexylbenzene was added to the solution as internal standard to estimate the yield. Gas chromatographic determination demonstrated that the yield of 2-trifluoromethylacrylic acid benzyl ester was 75 % based on 3,3,3-trifluoro-2-bromopropene as standard. From the reaction solution, the benzylchloride employed in excess and the solvent DMF was recovered by distillation. The residue which was then purified by chromatography with a silica gel column gave 6.2 g (or

0.027 mol) of 2-trifluoromethylacrylic acid benzyl ester.

Example 33

Procedure of the Reaction Step (I) was followed in the same manner as in Example 32.

To the reaction mixture of the Reaction Step (I) from which remaining zinc powder had been removed by filtration, 13.1 g (or 0.12 mol) of ethylbromide was mixed and the resulting mixture was placed in the autoclave to allow to react at 110° C under stirring for 3 hr. The reaction mixture thus prepared gave by distillation under a reduced pressure 4.8 g (or 0.029 mol) of 2-trifluoromethylacrylic acid ethyl ester with a yield of 71 %.

Example 34

The same procedure as in the Reaction Step (I) in Example 32 was followed.

To the reaction mixture of the Reaction Step (I) from which remaining zinc powder had been removed by filtration, 9.2 g (or 0.12 mol) of allylchloride was mixed and the resulting mixture was placed in the autoclave to allow to react at 60° C under stirring for 8 hr. The reaction mixture thus prepared gave by distillation under a reduced pressure 4.8 g (or 0.027 mol) of 2-trifluoromethylacrylic acid allyl ester with a yield of 67 %.

Example 35

Reaction Step (I)

The same procedure as that of the Reaction Step (I) in Example 31 was followed except that 37.2 g (or 0.1 mol) of 3,3,4,4,5,5,6,6,6-nonafluoro-2-iodohexene was employed in place of 3,3,3-trifluoro-2-iodopropene.

Reaction Step (II)

To the reaction mixture of the Reaction Step (I) from which remaining zinc powder had been removed, 32.7 g (or 0.3 mol) of ethylbromide was mixed and the resulting mixture was placed in the autoclave to allow to react at 110° C under stirring for 5 hr. The reaction mixture thus prepared gave by distillation under a reduced pressure 19.7 g (or 0.062 mol) of 2-perfluorobutylacrylic acid ethyl ester with a yield of 62 %.

Example 36

Reaction Step (I)

The same procedure as that in the Reaction Step (I) in Example 32 was followed except that 8.3 g (or 0.04 mol) of 1,2,2-trifluoro-1-iodoethene was employed in place of 3,3,3-trifluoro-2-bromopropene.

Reaction Step (II)

To the reaction mixture of the Reaction Step (I) from which remaining zinc powder had been removed, 17.0 g (or 0.12 mol) of methyliodide was mixed and the resulting mixture was again placed in the autoclave to allow to react at 60° C under stirring for 5 hr. The reaction mixture thus prepared gave by distillation 2.2 g (or 0.016 mol) of 1,2,2-trifluoroacrylic acid methyl ester with a yield of 40 %.

Example 37

The same procedure of the Reaction Step (I) as that of Example 36 was followed. To the reaction mixture of the Reaction Step (I) from which remaining zinc powder had been removed by filtration, 56.9 g (or 0.12 mol) of 1,1,2,2-tetrahydro-1-iodoperfluorooctane was mixed and the resulting mixture was again placed in the autoclave to allow to react at 80° C under stirring for 5 hr. The reaction mixture thus prepared gave by distillation 6.6 g (or 0.014 mol) of 1,1,2,2-tetrahydroperfluorooctyl ester of 1,2,2-trifluoroacrylic acid with a yield of 35 %.

Example 38

The Reaction Step (I) was carried out in the same manner as in Example 29. Then remaining zinc powder was removed by filtration from the reaction mixture. To this reaction mixture was added 112.2 g (or 0.3 mol) of 1,1,2,2-tetrahydro-1-iodoperfluorohexane and the subsequent treatment was the same as in the Reaction Step (II) in Example 29, to obtain 50.4 g (or 71 mmol) of 1,1,2,2-tetrahydroperfluorohexyl ester of perfluorononanoic acid.

Yield : 71 %,
Boiling point: 95 - 97 °C/6 mm Hg.

Example 39

The Reaction Step (I) was carried out in the same manner as in Example 19 and remaining zinc powder was removed by filtration from the reaction mixture. To this reaction mixture was added 224.4 g (or 0.6 mol) of 1,1,2,2-tetrahydro-1-iodoperfluorohexane. Subsequent treatment was the same as described in the Reaction Step (II) in Example 19, to obtain 57.9 g (or 74 mmol) of 1,1,2,2-tetrahydro-perfluorohexyl diester of perfluoroadipic acid. The yield was 74 %.

Example 40

The Reaction Step (I) was carried out in the same manner as in Example 32 and remaining zinc powder was removed by filtration from the reaction mixture. To this reaction mixture was added 44.9 g (or 0.12 mol) of 1,1,2,2-tetrahydro-1-iodoperfluorohexane. Subsequent treatment was the same as described in the Reaction Step (II) in Example 32, to obtain 4.6 g (or 12 mmol) of 1,1,2,2-tetrahydro-perfluorohexyl diester of $\alpha$-trifluoromethylacrylic acid. The yield was 30 %.

Table 5

| Example | $X-R_f-X'$ | R-Y | Product | Yield |
|---|---|---|---|---|
| 1 | $n-C_8F_{17}I$ | $C_2H_5Br$ | $n-C_8F_{17}COOC_2H_5$ | 90.1 |
| 2 | " | $CH_3I$ | $n-C_8F_{17}COOCH_3$ | 91.7 |
| 3 | $n-C_6F_{13}I$ | " | $n-C_6F_{13}COOCH_3$ | 91.7 |
| 4 | $n-C_4F_9I$ | " | $n-C_4F_9COOCH_3$ | 90.1 |
| 5 | $n-C_8F_{17}I$ | $n-C_3H_7l$ | $n-C_8F_{17}COOC_3H_7$ | 92.3 |
| 6 | " | $n-C_4H_9I$ | $n-C_8F_{17}COOC_4H_9$ | 90.2 |
| 7 | " | $(CH_3)_2CHCH_2CH_2I$ | $n-C_8F_{17}COOCH_2CH_2CH(CH_3)_2$ | 88.7 |
| 8 | Same as Example 1, and N-methylpyrrolidone, N,N-dimethylacetamide, dimethylsulfoxide were used as solvent. | | | 84.2 |
| 9 | | | | 86.7 |
| 10 | | | | 80.5 |
| 11 | $n-C_8F_{17}I$ | $CH_2=CHCH_2Cl$ | $n-C_8F_{17}COOCH_2CH=CH_2$ | 66.5 |
| 12 | " | $CH_2=CHCH_2Br$ | " | 79 |
| 13 | $n-C_4F_9I$ | $CH_2=CHCH_2Cl$ | $n-C_4F_9COOCH_2CH=CH_2$ | 68.3 |
| 14 | $n-C_8F_{17}I$ | $CH_2=CHCH(CH_3)Cl$ | $n-C_8F_{17}COOCH(CH_3)CH=CH_2$ | 65 |
| 15 | " | $CH_2=CHCH_2CH_2Br$ | $n-C_8F_{17}COOCH_2CH_2CH=CH_2$ | 77.1 |
| 16 | Same as Example 11, and N-methylpyrrolidone, N,N-dimethylacetamide, dimethylsulfoxide were used as solvent. | | | 62.1 |
| 17 | | | | 64.0 |
| 18 | | | | 59.4 |

Table 5 (Cont'd)

| Example | X-$R_f$-X' | R-Y | Product | Yield |
|---|---|---|---|---|
| 19 | $I(CF_2CF_2)_2I$ | $C_2H_5Br$ | $C_2H_5OOC(CF_2CF_2)_2COOC_2H_5$ | 88.3 |
| 20 | " | $CH_3I$ | $CH_3OOC(CF_2CF_2)_2COOCH_3$ | 68.6 |
| 21 | $I(CF_2CF_2)_3I$ | $C_2H_5Br$ | $C_2H_5OOC(CF_2CF_2)_3COOC_2H_5$ | 87.5 |
| 22 | $I(CF_2CF_2)_2I$ | $n\text{-}C_3H_7I$ | $n\text{-}C_3H_7OOC(CF_2CF_2)_2COO(n\text{-}C_3H_7)$ | 82.9 |
| 23 | " | $n\text{-}C_4H_9I$ | $n\text{-}C_4H_9OOC(CF_2CF_2)_2COO(n\text{-}C_4H_9)$ | 80.3 |
| 24 | " | $(CH_3)_2CHCH_2CH_2I$ | $(CH_3)_2CHCH_2CH_2OOC(CF_2CF_2)_2$ $-COOCH_2CH_2CH(CH_3)_2$ | 79.1 |
| 25 | " | $CH_2=CHCH_2Cl$ | $CH_2=CHCH_2OOC(CF_2CF_2)_2$ $-COOCH_2CH=CH_2$ | 62.3 |
| 26 | Same as Example 19, and N-methylpyrrolidone, N,N-dimethylacetamide, dimethylsulfoxide were used as solvent. | | | 82.3 |
| 27 | | | | 83.7 |
| 28 | | | | 79.8 |
| 29 | $C_8F_{17}I$ | 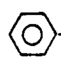$-CH_2Cl$ | $C_8F_{17}COOCH_2-$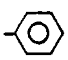 | 75 |
| 30 | $I(CF_2CF_2)_3I$ | 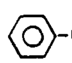$-CH_2Br$ | 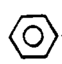$-CH_2OOC(CF_2CF_2)_3COOCH_2-$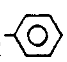 | 67.5 |
| 31 | $CH_2=C(CF_3)I$ | $CH_3I$ | $CH_2=C(CF_3)COOCH_3$ | 79 |
| 32 | $CH_2=C(CF_3)Br$ | 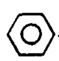$-CH_2Cl$ (Zinc and LiCl were present in the Reaction Step (I).) | $CH_2=C(CF_3)COOCH_2-$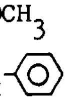 | 75 |
| 33 | " | $CH_3CH_2Br$ (Zinc and LiCl were present in the Reaction Step (I).) | $CH_2=C(CF_3)COOCH_2CH_3$ | 71 |
| 34 | $CH_2=C(CF_3)Br$ | $CH_2=CHCH_2Cl$ (Zinc and LiCl were present in the Reaction Step (I).) | $CH_2=C(CF_3)COOCH_2CH=CH_2$ | 67 |
| 35 | $CH_2=C(n\text{-}C_4F_9)I$ | $C_2H_5Br$ | $CH_2=C(n\text{-}C_4F_9)COOC_2H_5$ | 62 |
| 36 | $CF_2=CFI$ | $CH_3I$ | $CF_2=CFCOOCH_3$ | |
| 37 | " | $n\text{-}C_6F_{13}CH_2CH_2I$ | $CF_2=CFCOOCH_2CH_2C_6F_{13}$ | |

Table 5 (Cont'd)

| Example | $X\text{-}R_f\text{-}X'$ | R-Y | Product | Yield |
|---|---|---|---|---|
| 38 | $C_8F_{17}I$ | $C_4F_9CH_2CH_2I$ | $C_8F_{17}COOCH_2CH_2C_4F_9$ | 71 |
| 39 | $I(CF_2)_4I$ | $C_4F_9CH_2CH_2I$ | $C_4F_9CH_2CH_2O_2C(CF_2)_4CH_2CH_2C_4F_9$ | 74 |
| 40 | $CH_2{=}C(CF_3)Br$ | $C_4F_9CH_2CH_2I$ | $CH_2{=}C(CF_3)COOCH_2CH_2C_4F_9$ | 30 |

## Claims

1. A process for the preparation of a carboxylic acid ester containing fluorine characterised in that it comprises the reaction of a halogenated hydrocarbon represented by the general formula (A)

   (A) RY

   where R represents
   a straight or branched chained alkyl group having 1 to 20 carbon atoms which optionally contains fluorine,
   a saturated or unsaturated straight or branched chained aliphatic group having up to 20 carbon atoms which is substituted by an aromatic ring,
   or an alkenyl group represented by a general formula

   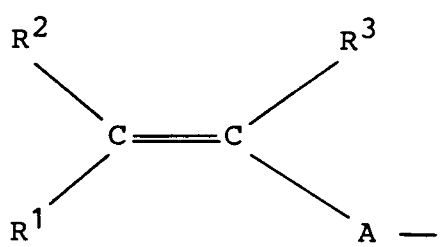

   where $R^1$, $R^2$ and $R^3$ independently represent
   a hydrogen atom
   or a straight or branched chain alkyl group
   and A represents a methylene group or a straight or branched chain alkylene group having 3 to 20 carbon atoms
   and Y represents a chlorine atom, a bromine atom or an iodine atom
   with a mixture obtainable by the reaction of an organic halogenated compound containing fluorine represented by the formula (B), with carbon dioxide in the presence of zinc:

   (B) $X - R_f - X'$

   where X and X′ independently represent
   fluorine, chlorine, bromine, or iodine atoms
   with the proviso that X and X′ may not both represent fluorine atoms
   and $R_f$ represents a saturated or unsaturated alphatic group which is straight or branched chains either having 1 to 20 carbon atoms when X or X′ represents a fluorine atom
   or having 3 to 20 carbon atoms when neither X nor X′ represents a fluorine atom.

2. A process according to claim 1 characterised in that the mixture obtainable by the reaction of the

organic halogen compound containing fluorine represented by the formula (B) with carbon dioxide in the presence of zinc is so obtained.

**3.** A process according to any one of the preceding claims characterised in that the reaction of the organic halogenated compound containing fluorine with carbon dioxide in the presence of zinc is carried out in the presence of one or more of the following cations:
alkali metal ions,
alkaline earth ions,
or ammonium ions

**4.** A process according to any one of the preceding claims characterised in that the organic halogenated compound containing fluorine is represented by the formula (C)

(C) $R_f$ X

where X is as defined for B, except that X does not represent a fluorine atom and
$R_f$ is a fluorine
containing aliphatic group having 1 to 20 carbon atoms.

**5.** A process according to claim 4 characterised in that X is bonded to a saturated carbon atom.

**6.** A process according to claim 4 characterised in that $R_f$ represents an unsaturated group having 2 to 20 carbon atoms and X is bonded to an unsaturated carbon atom.

**7.** A process according to any one of claims 1 to 3 characterised in that the organic halogenated compound containing fluorine is represented by the formula (B) where neither X nor X' represents a fluorine atom, and $R_f$ is a fluorine containing bifunctional group and each of X and X' is independently bonded to a saturated carbon atom.

**8.** A process according to any one of claims 1 to 7 characterised in that the organic halogenated compound containing fluorine is either
(i) a perfluoroalkyliodide represented by the general formula:

$C_nF_{2n+1}.I$

(where n represents an integer from 1 to 20),
(ii) a perfluoro alkylene diiodide having a straight or branched chain represented in the general formula:

$I.C_nF_{2n}.I$

(where n represents an integer from 3 to 20),
or (iii) an alpha-perfluoro alkyl substituted vinyl halogenide compound represented by the general formula:

$H_2C=C(C_nF_{2n+1}).X$

(where n represents an integer between 1 and 18 and X represents a bromine or iodine atom)

**9.** A process according to any one of the preceding claims characterised in that the halogenated hydrocarbon represented by the general formula (A) contains fluorine and that R represents an alkyl group having 1 to 20 carbon atoms.

**10.** A process according to any one of the preceding claims characterised in that the halogenated hydrocarbon represented by general formula (A) is an alkenyl compound represented by a general formula:

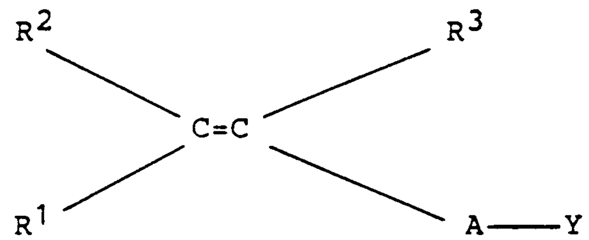

**11.** A process according to any one of the preceeding claims characterised in that the halogenated hydrocarbon is ethylbromide, methyliodide, N-propyliodide, n-butyliodide, 1-iodo-3-methylbutane, 3,3,4,4,5,5,6,6,7,7,8,8,8-tridecafluoro-1-iodooctane, 3,3,4,4,5,5,6,6,7,7,8,8,9,9,10,10,10-heptadecafluoro-1-iododecane, benzylchloride, benzylbromide, allyl chloride, allyl bromide, 3-chloro-1-butene, and 4-bromo-1-butene.

**Patentansprüche**

**1.** Verfahren zur Herstellung eines fluorhaltigen Carbonsäureesters, dadurch gekennzeichnet, daß es die Umsetzung eines halogenierten Kohlenwasserstoffs der allgemeinen Formel (A)

(A) RY

in der R einen gegebenenfalls fluorhaltigen gerad- oder verzweigtkettigen Alkylrest mit 1 bis 20 Kohlenstoffatomen,
einen gesättigten oder ungesättigten, gerad- oder verzweigtkettigen, mit einem aromatischen Ring substituierten aliphatischen Rest mit bis zu 20 Kohlenstoffatomen, oder
einen Alkenylrest der allgemeinen Formel

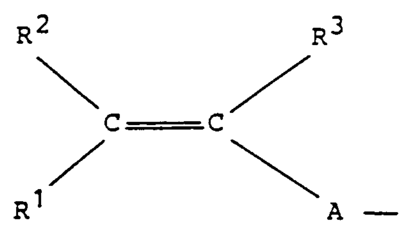

bedeutet, in der $R^1$, $R^2$ und $R^3$ unabhängig voneinander ein Wasserstoffatom oder einen gerad- oder verzweigtkettigen Alkylrest darstellen, und
A eine Methylengruppe oder einen gerad- oder verzweigtkettigen Alkylenrest mit 3 bis 20 Kohlenstoffatomen darstellt und
Y ein Chlor-, Brom-, oder Jodatom darstellt,
mit einem Gemisch umfaßt, das durch die Umsetzung einer fluorhaltigen organischen halogenierten Verbindung der Formel (B) mit Kohlendioxid in Gegenwart von Zink erhältlich ist:

(B) $X - R_f - X'$

in der X und X' unabhängig voneinander Fluor-, Chlor-, Brom- oder Jodatome bedeuten,
mit der Maßgabe, daß X und X' nicht gleichzeitig Fluoratome sein dürfen und
$R_f$ einen gesättigten oder ungesättigten aliphatischen, gerad- oder verzweigtkettigen Rest darstellt, der entweder 1 bis 20 Kohlenstoffatome besitzt, falls X oder X' ein Fluoratom bedeutet,
oder 3 bis 20 Kohlenstoffatome besitzt, wenn weder X noch X' ein Fluoratom darstellen.

**2.** Verfahren nach Anspruch 1, dadurch gekennzeichnet, daß das durch die Umsetzung einer fluorhaltigen organischen Halogenverbindung der Formel (B) mit Kohlendioxid in Gegenwart von Zink erhältliche Gemisch, so erhalten worden ist.

**3.** Verfahren nach einem vorangehenden Anspruch, dadurch gekennzeichnet, daß die Umsetzung der fluorhaltigen organischen halogenierten Verbindung mit Kohlendioxid in Gegenwart von Zink durchgeführt wird, in Gegenwart eines oder mehrerer der nachstehenden Kationen:
Alkalimetallionen,
Erdalkalimetallionen oder
Ammoniumionen.

**4.** Verfahren nach einem der vorangehenden Ansprüche, dadurch gekennzeichnet, daß die fluorhaltige organische halogenierte Verbindung durch die Formel (C)

(C) $R_f X$

wiedergegeben wird, in der X wie für B definiert ist, mit der Ausnahme daß X kein Fluoratom darstellt, und in der
$R_f$ einen fluorhaltigen aliphatischen Rest mit 1 bis 20 Kohlenstoffatomen bedeutet.

**5.** Verfahren nach Anspruch 4, dadurch gekennzeichnet, daß X an ein gesättigtes Kohlenstoffatom gebunden ist.

**6.** Verfahren nach Anspruch 4, dadurch gekennzeichnet, daß $R_f$ einen ungesättigten Rest mit 2 bis 20 Kohlenstoffatomen bedeutet und X an ein ungesättigtes Kohlenstoffatom gebunden ist.

**7.** Verfahren nach einem der Ansprüche 1 bis 3, dadurch gekennzeichnet, daß die fluorhaltige organische halogenierte Verbindung durch die Formel (B) wiedergegeben wird, in der weder X noch X' ein Fluoratom darstellen und $R_f$ einen fluorhaltigen bifunktionellen Rest bedeutet und jede der Gruppen X und X' unabhängig voneinander an ein gesättigtes Kohlenstoffatom gebunden ist.

**8.** Verfahren nach einem der Ansprüche 1 bis 7, dadurch gekennzeichnet, daß die fluorhaltige organische halogenierte Verbindung entweder
(i) ein Perfluoralkyljodid der allgemeinen Formel:

$C_nF_{2n+1}.I$

(in der n eine ganze Zahl von 1 bis 20 ist),
(ii) ein Perfluoralkylendijodid mit einer geraden oder verzweigten Kette der allgemeinen Formel:

$I.C_nF_{2n}.I$

(in der n eine ganze Zahl von 3 bis 20 ist), oder
(iii) eine $\alpha$-perfluoralkylsubstituierte Vinylhalogenidverbindung der allgemeinen Formel

$H_2C=C(C_nF_{2n+1}).X$ ist

(in der n eine ganze Zahl zwischen 1 und 18 ist und X ein Brom- oder Jodatom bedeutet).

**9.** Verfahren nach einem der vorangehenden Ansprüche, dadurch gekennzeichnet, daß der halogenierte Kohlenwasserstoff der allgemeinen Formel (A) Fluor enthält und daß R einen Alkylrest mit 1 bis 20 Kohlenstoffatomen bedeutet.

**10.** Verfahren nach einem der vorangehenden Ansprüche, dadurch gekennzeichnet, daß der halogenierte Kohlenwasserstoff der allgemeinen Formel (A) eine Alkenylverbindung ist, die durch die allgemeine Formel:

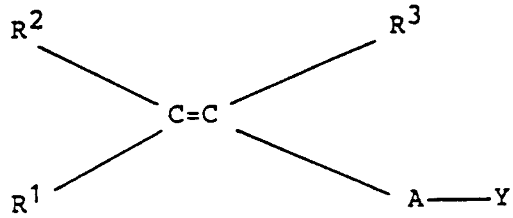

wiedergegeben wird.

**11.** Verfahren nach einem der vorangehenden Ansprüche, dadurch gekennzeichnet, daß der halogenierte Kohlenwasserstoff Ethylbromid, Methyljodid, N-Propyljodid, n-Butyljodid, 1-Jod-3-methylbutan, 3,3,4,4,5,5,6,6,7,7,8,8,8-Tridecafluor-1-jodoctan, 3,3,4,4,5,5,6,6,7,7,8,8,9,9,10,10,10-Heptadecafluor-1-joddecan, Benzylchlorid, Benzylbromid, Allylchlorid, Allylbromid, 3-Chlor-1-buten und 4-Brom-1-buten ist.

**Revendications**

**1.** Procédé pour la préparation d'un ester d'acide carboxylique contenant du fluor, caractérisé en ce qu'il comprend la réaction d'un hydrocarbure halogéné répondant à la formule générale (A)

(A) RY

dans laquelle R représente
un groupe alkyle à chaîne droite ou ramifiée ayant de 1 à 20 atomes de carbone, qui contient éventuellement du fluor,
un groupe aliphatique à chaîne droite ou ramifiée, saturé ou insaturé, pouvant avoir jusqu'à 20 atomes de carbone, qui est substitué par un cycle aromatique,
ou un groupe alcényle répondant à la formule générale :

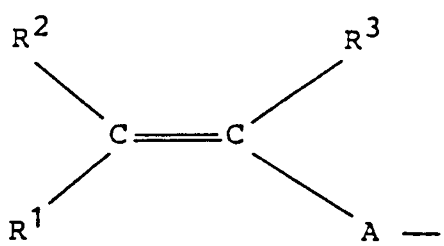

dans laquelle $R^1$, $R^2$ et $R^3$ représentent indépendamment un atome d'hydrogène
ou un groupe alkyle à chaîne droite ou ramifiée
et A représente un groupe méthylène ou un groupe alkylène à chaîne droite ou ramifiée ayant de 3 à 20 atomes de carbone
et Y représente un atome de chlore, un atome de brome ou un atome d'iode
avec un mélange que l'on peut obtenir par la réaction d'un composé organique halogéné contenant du fluor répondant à la formule (B), avec du dioxyde de carbone en présence de zinc:

(B) $X - R_f - X'$

sachant que X et X' représentent indépendamment
des atomes de fluor, de chlore, de brome, ou d'iode
à condition que X et X' ne puissent pas représenter tous les deux des atomes de fluor
et $R_f$ représente un groupe aliphatique saturé ou insaturé qui est à chaîne droite ou ramifiée
ayant de 1 à 20 atomes de carbone lorsque X ou X' représente un atome de fluor
ou ayant de 3 à 20 atomes de carbone lorsque ni X ni X' ne représente un atome de fluor.

**2.** Procédé selon la revendication 1, caractérisé en ce que l'on obtient de cette manière le mélange que

l'on peut obtenir par la réaction du composé organique halogéné contenant du fluor répondant à la formule (B) avec du dioxyde de carbone en présence de zinc.

**3.** Procédé selon l'une quelconque des revendications précédentes, caractérisé en ce que l'on met en oeuvre la réaction du composé organique halogéné contenant du fluor avec du dioxyde de carbone en présence de zinc, en présence d'un ou plusieurs des cations suivants :
des ions des métaux alcalins,
des ions alcalino-terreux,
ou des ions ammonium.

**4.** Procédé selon l'une quelconque des revendications précédentes, caractérisé en ce que le composé organique halogéné contenant du fluor, répond à la formule (C) :

(C) $R_fX$

dans laquelle X est tel qu'on l'a défini pour (B), excepté que X ne représente pas un atome de fluor et $R_f$ représente un groupe aliphatique contenant du fluor ayant de 1 à 20 atomes de carbone.

**5.** Procédé selon la revendication 4, caractérisé en ce que X est lié à un atome de carbone saturé.

**6.** Procédé selon la revendication 4, caractérisé en ce que $R_f$ représente un groupe insaturé ayant de 2 à 20 atomes de carbone, et X est lié à un atome de carbone insaturé.

**7.** Procédé selon l'une quelconque des revendications 1 à 3, caractérisé en ce que le composé organique halogéné contenant du fluor répond à la formule (B) dans laquelle ni X ni X' ne représente un atome de fluor, et $R_f$ est un groupe bifonctionnel contenant du fluor et chacun des groupes X et X' est indépendamment lié à un atome de carbone saturé.

**8.** Procédé selon l'une quelconque des revendications 1 à 7, caractérisé en ce que le composé organique halogéné contenant du fluor est :
(i) un iodure de perfluoroalkyle répondant à la formule générale :

$C_nF_{2n+1}.I$

(dans laquelle n représente un nombre entier compris entre 1 et 20),
(ii) un diiodure de perfluoroalkylène à chaîne droite ou ramifiée répondant à la formule générale :

$I.C_nF_{2n}.I$

(dans laquelle n représente un nombre entier compris entre 3 et 20),
ou (iii) un composé halogénure de vinyle substitué en alpha par un groupe perfluoroalkyle répondant à la formule générale :

$H_2C=C(C_nF_{2n+1}).X$

(dans laquelle n représente un nombre entier compris entre 1 et 18 et X représente un atome de brome ou d'iode).

**9.** Procédé selon l'une quelconque des revendications précédentes, caractérisé en ce que l'hydrocarbure halogéné répondant à la formule générale (A) contient du fluor et en ce que R représente un groupe alkyle ayant de 1 à 20 atomes de carbone.

**10.** Procédé selon l'une quelconque des revendications précédentes, caractérisé en ce que l'hydrocarbure halogéné répondant à la formule générale (A) est un composé alcénylique répondant à la formule générale :

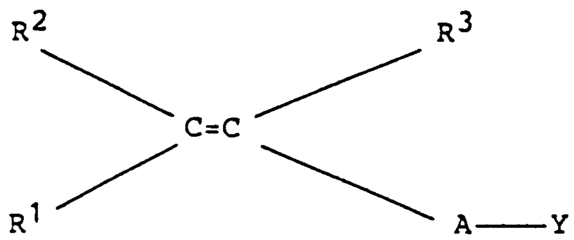

**11.** Procédé selon l'une quelconque des revendications précédentes, caractérisé en ce que l'hydrocarbure halogéné est le bromure d'éthyle, l'iodure de méthyle, l'iodure de n-propyle, l'iodure de n-butyle, le 1-iodo-3-méthylbutane, le 3,3,4,4,5,5,6,6,7,7,8,8,8-tridécafluoro-1-iodooctane, le 3,3,4,4,5,5,6,6,7,7,8,8,9,9,10,10,10-heptadécafluoro-1-iododécane, le chlorure de benzyle, le bromure de benzyle, le chlorure d'allyle, le bromure d'allyle, le 3-chloro-1-butène, et le 4-bromo-1-butène.